(19) Europäisches Patentamt
European Patent Office
Office européen des brevets

(11) Publication number: **0 160 507 B1**

(12) **EUROPEAN PATENT SPECIFICATION**

(45) Date of publication of patent specification: **04.07.90**

(51) Int. Cl.$^5$: **C 11 D 1/88,** C 11 D 1/94, A 61 K 7/075

(21) Application number: **85302876.9**

(22) Date of filing: **24.04.85**

(54) **Shampoo compositions.**

(30) Priority: **25.04.84 GB 8410503**

(43) Date of publication of application:
**06.11.85 Bulletin 85/45**

(45) Publication of the grant of the patent:
**04.07.90 Bulletin 90/27**

(84) Designated Contracting States:
**AT BE CH DE FR GB IT LI LU NL SE**

(56) References cited:
**EP-A-0 162 600**
**FR-A-2 204 397**
**GB-A-2 023 637**
**US-A-3 341 460**

(73) Proprietor: **Fishlock-Lomax, Eric Graham**
**Douglas House**
**Chipping Warden Nr. Banbury Oxfordshire (GB)**

(72) Inventor: **Fishlock-Lomax, Eric Graham**
**Douglas House**
**Chipping Warden Nr. Banbury Oxfordshire (GB)**

(74) Representative: **Skailes, Humphrey John et al**
**Frank B. Dehn & Co. Imperial House 15-19**
**Kingsway**
**London WC2B 6UZ (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 160 507 B1

Courier Press, Leamington Spa, England.

**Description**

This invention relates to shampoo compositions which are intended for frequent use and which contain a hair conditioner which does not build up on the hair.

Conditioning agents currently used are usually cationic surfactants such as stearyl dimethyl benzyl ammonium chloride, cetrimide, or other quaternary ammonium compounds, or cationic polymers i.e. polymers containing a multiplicity of cationic groups such as quaternary ammonium groups. Betaines may also be used for this purpose. These types of conditioning agents are strongly substantive to the hair and, whilst they are ideal for occasional use, they have the disadvantage when used frequently of building up on the hair with the undesirable effect of making the hair so greasy more easily and more quickly. This is the opposite of the desired effect and can give the hair a lank appearance.

One of the objects of this invention is to provide a combined shampoo and conditioning product which is a very mild to eyes and skin. Another object of this invention is to provide such a product which does not build up on the hair to cause undesirable effects.

The invention thus provides a shampoo composition containing at least two amphoteric surfactants of the general formula (I)

$$R{-}(CO)_z{-}(OCH_2CH_2CH_2)_n{-}(\underset{\displaystyle B}{\underset{|}{N}}{-}(CHR^1)_x)_y{-}\underset{\displaystyle B}{\underset{|}{N}}{-}Q \qquad (I)$$

where R is a $C_{8-20}$ hydrocarbyl group, optionally substituted;

$R^1$ is H or $C_{1-6}$ alkyl;

B is H, alkyl or substituted alkyl, or a group Q as defined below;

Q is an anionic moiety;

x is 2 to 6; and

n is 0 or 1 (z being 0 when n is 1);

the composition containing a first type of amphoteric surfactant of the above formula (in which z=1 and y is 0 or 1) which acts as a low or non-irritant cleaner, and a second type (in which z=0 and y is 1 to 5) which primarily functions as a conditioner. The composition also includes an anionic surfactant.

In the compounds of formula (I), R may for example be a straight or branched alkyl or alkenyl group; a cycloalkyl-alkyl (e.g. cyclohexyl-alkyl) group; an aralkyl or aralkenyl group in which the alkyl or alkenyl portion contains at least 6 carbon atoms; or the hydrocarbyl portion of a resinic acid containing at least two fused rings, e.g. as in the tricyclic pine resin acids such as abietic acid.

R is preferably a $C_{10-16}$ alkyl group, e.g. a $C_{12}$ group and an example of a branched chain group is $C_{16}H_{33}CH_2(CH_3)$—. The aliphatic portion of R may for example be the hydrocarbyl portion of lauric or coconut fatty acid, both of which contain a high proportion of $C_{12}$ constituents. An example of an unsaturated R group is oleyl. R may for example be substituted by hydroxy, as in hydroxystearyl or by —COOH (e.g. at the 2-position).

In most surfactants of the formula (I) type, n is 0.

$R^1$ is usually a hydrogen atom, but may be an alkyl group such as methyl.

When B is an alkyl group, it may have 1—6, preferably 2—4, carbon atoms, and is preferably a straight chain group. Examples of such groups are methyl and ethyl. The alkyl group may be substituted, for example by hydroxy, as in 2-hydroxymethyl, or by amino.

The group Q may for example be of the formula —$R^2$COOM where $R^2$ is a $C_{1-6}$ alkylene group (such as methylene or ethylene) and M is hydrogen or an alkali metal, alkaline earth metal, ammonium or substituted ammonium ion (e.g. mono-, di- or tri-hydroxyethylammonium). M is preferably sodium, and $R^2$ is preferably methylene.

In the first (cleaning) type of amphoteric surfactant, x is preferably 2 and y is preferably 1.

The first type of amphoteric surfactant is thus preferably a compound of formula (I) in which R is a $C_{8-20}$ hydrocarbyl group, preferably derived from coco, oleic or tall oil fatty acid,

$R^1$ is H,

B is H, hydroxyethyl or a group Q as defined below,

Q is an anionic moiety, preferably —$CH_2COONa$ or —$CH_2CH_2COONa$,

x is 2,

y is 1; and

z is 1

Examples of suitable materials of this type are those known as cocoamphocarboxyglycinate and cocoamphoglycinate.

The second (conditioning) type of amphoteric surfactant may for example have the general formula (I) in which z is 0, x is 2 or 3 (preferably 2) and y is 1 or 2 to 5. The material preferably contains 3 or more nitrogen atoms and more preferably 4 or more nitrogen atoms; thus y is preferably 2 or 3 or more. The group R in the second type is preferably a $C_{16-18}$ alkyl group, $R^1$ is preferably H, Q is preferably —$CH_2COONa$ or —$CH_2CH_2COONa$, and B is preferably H or Q.

An example of this type of surfactant is Ampholak® QTE.

The compositions will generally contain from 95—50% (preferably 95—75%) of the first type of amphoteric surfactant and 5—50% (preferably 5—25%) of the second type, based on the total weight of the two materials (all percentages herein are by weight; amphoteric surfactants are normally supplied on a 30% active basis relative to undiluted material).

The shampoos also contain an anionic surfactant, which may be any kind suitable for use in shampoos. Examples of suitable materials are sodium $C_{9-15}$ alkyl sulphates or ether sulphates (containing for example 1—4 moles ethylene oxide/mole) and the corresponding ammonium, mono-, di- and tri-ethanolamine salts. The anionic surfactant is preferably sodium lauryl sulphate or a sodium lauryl ether sulphate containing 1—4 (preferably 2 or 3) moles of ethylene oxide. (Anionic surfactants of this type are normally supplied on a 27—30% active basis).

In general, the amount of anionic surfactant used may be such that the total amphoteric: anionic surfactant is from 3:1 to 1:5, preferably about 1:1, and examples of suitable compositions are:

| | | | |
|---|---|---|---|
| First type of amphoteric | 5—50%, | preferably | 5—25% |
| Second type of amphoteric | 1—50%, | preferably | 1—5% |
| Anionic | 5—50%, | preferably | 5—25% |

The shampoos of the invention are water-based and if desired may contain additional ingredients such as normally included in shampoos, for example preservatives, foam boosters (such as lauric diethanolamide or amine oxide), oils (e.g. jojoba or lemon), dyes and fragrances.

The following example illustrates the invention.

Example 1

A shampoo was prepared according to the following formula:

| | |
|---|---|
| Ampholak XCO-30 (first type above) (30% active) | 85 parts |
| Ampholak QTE (second type above) (30% active) | 15 parts |
| Sodium lauryl ether sulphate (27% active) | 100 parts |
| Water | 200 parts |

The pH of the above formulation was adjusted to about 6.5 with hydrochloric acid.

Subjective evaluation by a group of people showed that the shampoo gave excellent conditioning properties and the softness of the hair was particularly remarked upon. Since softness is an opposing property to the property shown after conditioner build up, this object of the invention was achieved.

The formulation was used regularly by the group and no undesirable properties were found even when used for many days consecutively.

Properties of mildness to eyes and skin were tested and found to be equivalent to the properties of a good baby shampoo, fulfilling another object to this invention.

Surprisingly other properties were found to be improved also. The foaming property of the formulation was found to be about 20% higher than that where only the first type of amphoteric surfactant was used. Also the viscosity of the shampoo was found to be very suitable without the need to add sodium chloride as a thickener.

**Claims**

1. A shampoo composition containing at least two amphoteric surfactants of the general formula (I)

$$R—(CO)_z—(OCH_2CH_2CH_2)_n—(\underset{\displaystyle B}{\underset{|}{N}}—(CHR^1)_x)_y—\underset{\displaystyle B}{\underset{|}{N}}—Q \qquad (I)$$

where R is a $C_{8-20}$ hydrocarbyl group, optionally substituted;

$R^1$ is H or $C_{1-6}$ alkyl;

B is H, alkyl or substituted alkyl, or a group Q as defined below;

Q is an anionic moiety;

x is 2 to 6; and

n is 0 or 1 (z being 0 when n is 1);

the composition containing a first amphoteric surfactant in which z is 1 and y is 0 or 1, and a second amphoteric surfactant in which z is 0 and y is 1 to 5, and the composition also containing at least one anionic surfactant.

2. A composition according to Claim 1 in which x is 2 in the first amphoteric surfactant and x is 2 or 3 in the second amphoteric surfactant.

3. A composition according to Claim 2 which contains:

(a) a first amphoteric surfactant in which R is derived from coco, oleic or tall oil fatty acid,

$R^1$ is —H

B is —H, hydroxyethyl or a group Q as defined below,
Q is $—CH_2COONa$ or $—CH_2CH_2COONa$,
x is 2,
y is 1, and
z is 1, and
(b) a second amphoteric surfactant in which
R is a $C_{16-18}$ alkyl group,
z is 0,
$R^1$ is —H,
B is —H or group Q as defined below,
Q is $—CH_2COONa$ or $—CH_2CH_2COONa$,
x is 2 or 3, and
y is 2 to 5.

4. A composition according to any preceding Claim which contains 95—50% of surfactant(s) in which z is 1 and 5—50% of surfactant(s) in which z is 0, by weight based on the total weight of said surfactants of formula (I).

5. A composition according to any preceding claim in which the total amphoteric surfactant to anionic surfactant is from 3:1 to 1:5, by weight.

6. A composition according to Claim 5 which contains 5—25% of an amphoteric surfactant in which z is 1, 1—5% of an amphoteric surfactant in which z is 0, and 5—25% of an anionic surfactant, by weight.

**Patentansprüche**

1. Shampoozusammensetzung, enthaltend zumindest zwei amphotere oberflächenaktive Mittel der allgemeinen Formel (I)

$$R—(CO)_z—(OCH_2CH_2CH_2)_n—(\underset{\displaystyle B}{\underset{|}{N}}—(CHR^1)_x)_y—\underset{\displaystyle B}{\underset{|}{N}}—Q \qquad (I)$$

worin R für eine gegebenenfalls substituierte $C_{8-20}$-Kohlenwasserstoffgruppe steht;
$R^1$ H oder $C_{1-6}$-Alkyl ist;
B H, Alkyl oder substituiertes Alkyl, oder eine wie nachstehend definierte Gruppe Q bedeutet;
Q ein anionischer Teil ist;
x für 2 bis 6 steht; und
n 0 oder 1 ist (wobei z 0 ist, wenn n für 1 steht);
wobei die Zusammensetzung ein erstes amphoteres oberflächenaktives Mittel, in dem z 1 ist, und y für 0 oder 1 steht, und ein zweites amphoteres oberflächenaktives Mittel, in dem z 0 ist, und y für 1 bis 5 steht, enthält, und die Zusammensetzung auch zumindest ein anionisches oberflächenaktives Mittel enthält.

2. Zusammensetzung gemäß Anspruch 1, in der x in dem ersten amphoteren oberflächenaktiven Mittel für 2 steht, und x in dem zweiten amphoteren oberflächenaktiven Mittel für 2 oder 3 steht.

3. Zusammensetzung gemäß Anspruch 2, die enthält
(a) ein erstes amphoteres oberflächenaktives Mittel, in dem sich R von Kokos-, Öl- oder Tallölfettsäure ableitet,
$R^1$ H ist,
B für -H, Hydroxyethyl oder eine wie nachstehend definierte Gruppe Q steht,
Q $—CH_2COONa$ oder $—CH_2CH_2COONa$ bedeutet,
x für 2 steht,
y für 1 steht, und
z für 1 steht, und
(b) ein zweites amphoteres oberflächenaktives Mittel, in dem
R eine $C_{16-18}$-Alkylgruppe bedeutet,
z für 0 steht,
$R^1$ —H ist,
B —H oder eine wie nachstehend definierte Gruppe Q ist,
Q für $—CH_2COONa$ oder $—CH_2CH_2COONa$ steht,
x für 2 oder 3 steht, und
y für 2 bis 5 steht.

4. Zusammensetzung gemäß einem der vorhergehenden Ansprüche, die 95—50% oberflächenaktive(s) Mittel, worin z für 1 steht, und 5—50% oberflächenaktive(s) Mittel, worin z für 0 steht, ausgedrückt als Gewicht, bezogen auf das Gesamtgewicht der oberflächenaktiven Mittel der Formel (I), enthält.

5. Zusammensetzung gemäß einem der vorhergehenden Ansprüche, in der das gesamte amphotere oberflächenaktive Mittel in Bezug auf das anionische oberflächenaktive Mittel sich wie 3:1 bis 1:5, auf das Gewicht bezogen, verhält.

6. Zusammensetzung gemäß Anspruch 5, die 5—25% amphoteres oberflächenaktives Mittel, worin z für 1 steht, 1—5% oberflächenaktives Mittel, worin z für 0 steht, und 5—25% anionisches oberflächenaktives Mittel, auf das Gewicht bezogen, enthält.

**Revendications**

1. Composition de shampooing contenant au moins deux surfactifs amphotères de formule générale (I)

$$R{-}(CO)_z{-}(OCH_2CH_2CH_2)_n{-}(\underset{\underset{B}{|}}{N}{-}(CHR^1)_x)_y{-}\underset{\underset{B}{|}}{N}{-}Q \qquad (I)$$

dans laquelle R est une groupe hydrocarboné en $C_8$—$C_{20}$, éventuellement substitué;
$R^1$ est H ou un alkyle en $C_{1-5}$;
B est H, alkyle ou alkyle substitué, ou un groupe Q comme défini ci-dessous;
Q est un reste anionique;
x est 2 à 6;
n est 0 ou 1 (z étant 0 quand n est 1);
la composition contenant un premier surfactif amphotère dans lequel z est 1 et y est 0 ou 1, et un second surfactif amphotère dans lequel z est 0 et y est 1 à 5, la composition contenant aussi au moins un surfactif anionique.

2. Composition selon la revendication 1, dans laquelle x est 2 dans le premier surfactif amphotère et x est 2 ou 3 dans le second surfactif amphotère.

3. Composition selon la revendication 2, qui contient:
(a) un premier surfactif amphotère dans lequel R dérive d'un acide gras d'huile de coprah, oléique ou de tall oil.
$R^1$ est —H
B est —H, hydroxyéthyle ou un groupe Q comme défini ci-dessous,
Q est —$CH_2COONa$ ou —$CH_2CH_2COONa$,
x est 2,
y est 1, et
z est 1, et
(b) un second surfactif amphotère dans lequel
R est un groupe alkyle en $C_{16}$—$C_{18}$,
z est 0,
R' est —H,
B est —H ou un groupe Q comme défini ci-dessus,
Q est —$CH_2COONa$ ou —$CH_2CH_2COONa$,
x est 2 ou 3, et
y est 2 à 5.

4. Composition selon l'une quelconque des revendications précédentes, qui contient 95—50% de surfactif(s) dans le(s)quel(s) z est 1 et 5—50% de surfactif(s) dans le(s)quel(s) z est 0, en poids par rapport au poids total desdits surfactifs de formule (I).

5. Composition selon l'une quelconque des revendications précédentes, dans laquelle la proportion de surfactif amphotère totale au surfactif anionique est de 3:1 à 1:5 en poids.

6. Composition selon la revendication 5, qui contient 5—25% d'un surfactif amphotère dans lequel z est 1, 1—5% d'un surfactif amphotère dans lequel z est 0, et 5—25% d'un surfactif anionique, en poids.